# NEUE EUROPÄISCHE PATENTSCHRIFT

(11) **EP 2 067 492 B2**
(45) Veröffentlichungstag und Bekanntmachung des Hinweises auf die Entscheidung über den Einspruch: **10.12.2025**
(45) Hinweis auf die Patenterteilung: 20.06.2018
(21) Anmeldenummer: 07022915.8
(22) Anmeldetag: 27.11.2007
(51) Int. Cl.: A61F 13/02, A61L 15/42, A61L 15/60

(54) **Polyacrylatpartikeln und ihre Verwendung**
Wound dressing with polyacrylate particles and its application
Pansement doté de particules de polyacrylate et son utilisation

(43) Veröffentlichungstag der Anmeldung: 10.06.2009
(73) Patentinhaber: Paul Hartmann AG, 89522 Heidenheim (DE)
(72) Erfinder: Smola, Hans, 50678 Köln (DE)
(74) Vertreter: DREISS Patentanwälte PartG mbB

(56) Entgegenhaltungen:
- EP-A1- 1 749 508
- EP-A2- 1 029 886
- WO-A1-2006/069732
- WO-A1-2007/041075
- WO-A1-99/16812
- WO-A1-99/57201
- WO-A2-2007/024972
- WO-A2-98/28013
- GB-A- 2 433 205
- JP-A- 2005 102 790
- US-A1- 2003 065 296
- US-A1- 2006 247 377
- H. SMOLA ET AL.: "Polyacrylatesuperabsorber inhibitis MMP activity in chronic wound fluid", WOUND REPAIR AND REGENERATION, vol. 15, no. 3, May 2007 (2007-05-01), pages A101

## Beschreibung

Die Erfindung betrifft Partikelgemische aus Polyacrylatpartikeln zum Einsatz in der modernen Wundbehandlung.
Die Heilung von Hautwunden beruht auf der Fähigkeit der Haut zur Regeneration von Epithelen sowie von Binde- und Stützgewebe. Sie ist als ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).
Zur Unterstützung der einzelnen Wundheilungsphasen sind zahlreiche Vorschläge in der Literatur beschrieben. Insbesondere sind auch Wundauflagen mit Polyacrylatpartikel seit geraumer Zeit Gegenstand zahlreicher Artikel in der Fachliteratur als auch von Patentschriften. So wird beispielsweise mit der US-Patentschrift US 5,977,428 A eine absorbierende Wundauflage beschrieben, die getrocknete, absorbierende Hydrogelpartikel innerhalb einer porösen Hülle umfasst. Diese Hydrogelpartikel können aus Polyacrylat bestehen, werden als Granulat oder Pulver eingesetzt und liegen innerhalb einer die Wundauflage bildenden Tasche aus einem textilen Material oder einem Nonwoven eventuell gemischt mit einem Bindungsmittel vor. Weiterhin wird mit der US-Patentschrift US 7,230,154 B2 eine Schaumwundauflage beschrieben, die eine haftende Wundkontaktschicht aus Silikon aufweist. In den Schaumkörper sind Polyacrylatpartikel eingebracht, die eine definierte Korngröße von 100 bis 900 µm aufweisen können. Mit der europäische Patentanmeldung EP 1688109 A1 wird eine Wundauflage zur Behandlung von trophischen Störungen in der Wunde beschrieben. Hierbei wird Kohlendioxid als Behandlungsmittel eingesetzt. In der Wundauflage ist ein Depot für eine wässrige Phase eingesetzt, das Polyacrylatpartikel enthält, die eine definierte Partikelverteilung aufweisen, wobei der Anteil an Partikel mit einer Partikelgröße von 850 bis 300 µm insbesondere mehr 70 Gew.-% bezogen auf das Gesamtgewicht der Partikel betragen soll. Diesen Schriften ist gemeinsam, das Polyacrylatpartikel immer als Speichermedium oder Absorptionsmedium für wässrige Flüssigkeiten verwendet wird.
WO2006/069732 offenbart beschichtete Partikel aus einem superabsorbierenden Polymer (SAP), die unter anderem für Pflaster zur Behandlung von Brandwunden eingesetzt werden können.
US2003/065296 beschreibt ein absorbierendes Material aus SAP und einem polymeren thermoplastischen Harz, welches unter anderem auch für Wundauflagen eingesetzt werden kann.
WO99/57201 offenbart gleichfalls eine Zusammensetzung, die eine thermoplastische Substanz und SAP enthalten kann. WO99/16812 offenbart ein absorbierendes Gelmaterial aus einem quervernetztem Polymer und einer Matrix. Das Gel kann unter anderem auch zur Wundbehandlung eingesetzt werden. Dabei umfasst es vorzugsweise kleine Partikel, d.h. mindestens 60 % der Partikel sollen eine Teilchengröße von weniger als 200 µm aufweisen.
US2006/247377 offenbart spezielle, insbesondere quervernetzte Acrylsäureester, welche unter anderem als absorbierendes Material bei Wundauflagen Verwendung finden können. Dazu werden Gele aus den quervernetzten Estern getrocknet und vermahlen, wobei Partikel in einem Größenbereich von 45 bis 1000 µm entstehen können. EP1749508 offenbart ein flüssigkeitsabsorbierendes Material mit einer Partikelgröße von weniger als 40 µm, welches in Verbindung mit einem inerten Träger vorliegt.
WO2007/024972 offenbart einen Wundverband, welcher sowohl nicht-freisetzbare, als auch kontrolliert freisetzbare antimikrobielle und Protease-inhibierende Substanzen enthält. Die kontrollierte Freisetzung der freisetzbaren Komponenten wird durch eine anionische Substanz bewirkt, bei der es sich auch um Polyacrylat handeln kann.
JP200510279 beschreibt ein Wundverbandmaterial zur Behandlung von Wunden wie Dekubitus mit einer Chitosan enthaltenden Wundkontaktschicht und einer stark flüssigkeitsabsorbierenden Polymerschicht, welches in der Lage ist, Wundflüssigkeit aufzunehmen und die Granulation durch Feuchthalten der Wunde zu beschleunigen.

Daher ist es eine Aufgabe der vorliegenden Erfindung ein verbessertes Mittel zur Behandlung von Wunden bereitzustellen. Darüber hinaus soll eine Wundauflage bereitgestellt werden, die den pathologischen Zustand einer Wunde derart beeinflusst, dass ein normaler, natürlicher Wundheilungsverlauf stattfinden kann.

Gelöst wird die Aufgabe durch ein Partikelgemisch gemäß Anspruch 1, das zur Verwendung bei der Behandlung von Dekubitus, Ulcus cruris venosum, Ulcus cruris arteriosum oder diabetischem Fußsyndrom eingesetzt wird, wobei das Partikelgemisch a) 20 bis 98 Gew.-% Partikel mit einer Partikelgröße x mit 45 ≤ x ≤ 300 µm und b) 2 bis 80 Gew.% Partikel mit einer Partikelgröße x mit x >300 µm umfasst. Ein erfindungsgemäßes Partikelgemisch umfasst eine definierte Menge einer ersten Partikelfraktion mit einer definierten Partikelgröße und eine zweite Menge einer zweiten Partikelfraktion mit einer von der ersten Partikelgröße verschiedenen zweiten Partikelgröße, wobei jede Partikelfraktion Polyacrylatpartikel enthält. Hierbei können sich die Polyacrylatpartikel eines Größenbereichs aus Partikeln gleicher oder unterschiedlicher Größe zusammensetzen, wobei die Partikelgröße(n) innerhalb des Bereichs liegt/ liegen. Im Zusammenhang mit der vorliegenden Erfindung wird dabei die Angabe einer Menge Partikel insbesondere eines einzelnen Größenbereichs - falls nicht anders angegeben - immer in Gewichts-% (Gew.-%) bezogen auf die Gesamtmenge an Partikeln angegeben.
Zudem kann auch vorgesehen sein, dass das Partikelgemisch Polyacrylatpartikel enthält, die sich hinsichtlich der Polyacrylate voneinander unterscheiden, d.h., dass das Partikelgemisch mindestens zwei voneinander verschiedene Polyacrylatpartikelsorten umfasst. Insbesondere kann auch vorgesehen sein, dass sich die Polyacrylatpartikel des ersten Größenbereichs a) von den Poyacrylatpartikeln des zweiten Größenbereichs b) unterscheiden. Die Polyacrylate können sich beispielsweise hinsichtlich des Neutralisationsgrades, des Vernetzungsgrades, des Vernetzungsmittels und/ oder der Copolymere unterscheiden. Im einfachsten Fall können jedoch auch Polyacrylatpartikel verwendet werden, die hinsichtlich ihres strukturellen Aufbaus gleich sind und lediglich in den angegebenen Partikelgrößen verschieden sind. Es kann jedoch auch vorgesehen sein, dass die Partikel des ersten oder des zweiten Partikelgrößenbereichs aus verschiedenen Polyacrylatpartikeln besteht, d.h., dass beispielsweise die Partikel des ersten Größenbereichs erste Polyacrylatpartikel und zweite, von den ersten verschiedene Polyacrylatpartikel enthält, wobei beide Sorten Polyacrylatpartikel umfassen, die innerhalb des jeweiligen Größenbereichs liegen.
Dabei soll im Zusammenhang mit der vorliegenden Erfindung unter einem Partikelgemisch ein Gemenge verstanden sein, deren einzelne Bestandteile (Partikel) räumlich neben einander, teilweise durchmischt, vollständig durchmischt oder räumlich getrennt voneinander vorliegen können, wobei das Gemisch in jedem Fall als Bestandteil einer Wundauflage anzusehen ist. Insbesondere können dabei die Partikel der einzelnen Partikelgrößenbereiche auch räumlich nebeneinander, teilweise durchmischt, vollständig durchmischt oder räumlich getrennt voneinander vorliegen.

Die Partikelgröße wird im Zusammenhang mit der vorliegenden Erfindung analog EDANA 420.2-02 bestimmt, wobei die Siebe (Durchmesser 200 mm) den Angaben entsprechende Lochgrößen aufweisen. Darüber hinaus können auch Siebe mit anderen Lochgrößen wie z.B. 125 µm, 160 µm, 630 µm, 900 µm, und 1500 µm verwendet werden. Hierbei werden trockene Polyacrylatpartikel mit einem Feuchtigkeitsgehalt von weniger als 10 Gew.-% Wasser bezogen auf das Gesamtgewicht der Partikel zugrunde gelegt, wobei der Feuchtigkeitsgehalt gemäß EDANA 450.2-02 bestimmt wird.

Überraschenderweise hat sich gezeigt, dass Polyacrylatpartikel Proteasen über diffusible Mechanismen inhibieren als auch durch direkte Bindung kompartmentieren und damit dem Wundexsudat bzw. der Wunde entziehen können. Insbesondere hat sich gezeigt, dass Polyacrylatpartikel zur Inhibition von Proteasen in chronischen Wunden geeignet sind. Zudem hat sich gezeigt, dass Metallo-Proteasen durch Polyacrylatpartikel gebunden oder kompartimentiert werden, so dass diese Metallo-Proteasen mit den Polyacrylatpartikeln aus einer Wundflüssigkeit oder einer Wunde entfernt werden können. Somit kann mittels der Polyacrylatpartikel ein Überschuss an Metallo-Proteasen in chronischen Wunden derart abgefangen werden, dass ein natürlicher Heilungsverlauf stattfinden kann. Hierfür sind überraschenderweise Polyacrylatpartikel mit einer Partikelgröße x mit x ≤ 300 µm besonders gut geeignet. Partikel dieser Größe hemmen die Aktivität von wundheilungsschädlichen Proteasen, insbesondere Metalloproteasen in einer Wunde besonders wirkungsvoll, wobei Partikel dieser Größe im Vergleich zu größeren Partikeln eine mindestens viermal so hohe Affinität zu Metalloproteasen aufweisen. Damit sind Polyacrylatpartikel mit einer Partikelgröße x mit x ≤ 300 µm besonders gut zur Behandlung von chronischen Wunden geeignet. Übliche Wundauflagen mit Polyacylatpartikeln zur Behandlung von chronischen Wunden enthalten Polyacrylatpartikel mit einer Partikelgröße x mit x > 300 µm, da diese Partikel meist gut zur Aufnahme von Wundexsudat oder als Speichermedium für wässrige Flüssigkeiten befähigt sind. Polyacrylatpartikel mit einer Partikelgröße x mit x ≤ 300 µm sind zur Aufnahme von Wundexsudat weniger gut oder gar nicht geeignet, da diese Partikel im Vergleich zu Partikeln mit einer Partikelgröße x mit x > 300 µm eine sehr viel geringere Menge an wässrigen Flüssigkeiten absorbieren oder halten können. Somit kann mit der vorliegenden Wundauflage aufgrund der spezifischen Auswahl von einem Partikelgemisch mit Partikeln definierter Größen ein Wundbehandlungsmittel bereitgestellt werden, das sowohl wundheilungshemmende Proteasen in einer Wunde inaktiviert als auch hydroaktive Eigenschaften bereitstellen kann.

Ein Überschuss an insbesondere Metallo-Proteasen ist insbesondere in der Granulationsphase der Wundheilung störend, da durch das gestörte Gleichgewicht von Metallo-Proteasen zu neu aufgebauten Bindegewebe kein ausreichender Gewebeaufbau stattfinden kann. Der Gewebeaufbau ist dabei kritisch von einer Stabilisierung neu synthetisierter extrazellulären Matrix-Moleküle abhängig, die zu einem dreidimensionalen Gewebe zusammengebaut werden, worauf parallel und in weiteren Schritten die Angiogenese und die Proliferation von Zellen in das Wundareal erfolgt.

Es hat sich nun gezeigt, dass Polyacrylatpartikel mit einer Partikelgröße mit x ≤ 300 µm insbesondere bei schlecht heilenden Wunden in der Granulationsphase eingesetzt werden können. In dieser Phase sind eventuelle Entzündungen soweit abgeklungen oder nicht vorhanden, dass eine Granulation und damit ein Zellwachstum stattfinden könnten, sofern kein Überschuss an Metallo-Proteasen vorliegt. Dieser Überschuss kann nun mittels Polyacrylatpartikeln der angegebenen Größe besonders gut abgefangen werden. Somit wird der Gewebeaufbau durch die Ausschaltung der die Wundheilung störenden Mechanismen gefördert, was sich klinisch in einer Besserung des Wundzustandes sowie der Wundverkleinerung durch Abnahme des Wundvolumens und/ oder der Wundgröße bemerkbar macht. Damit ist die Verwendung von Polyacrylatpartikeln mit einer Partikelgröße x mit x ≤ 300 µm zur Hemmung oder Inhibition von Proteasen insbesondere Serin-Proteasen oder Metallo-Proteasen zum Gewebeaufbau und/ oder zur Regulierung des Gewebeaufbaus in chronischen Wunden ebenfalls Gegenstand der vorliegenden Erfindung.

Im Zusammenhang mit der vorliegenden Erfindung sollen unter Proteasen (Peptidasen, Peptid-Hydrolasen) Enzyme verstanden sein, welche die hydrolytische Spaltung einer Peptid-Bindung in Proteinen und Peptiden (Proteolyse) katalysieren. Proteasen gehören damit systematisch zu der Gruppe der Hydrolasen. Proteasen werden hinsichtlich des Spaltungsortes im Substrat unterteilt. So wird die Spaltung von Peptidbindungen im Inneren von Peptiden oder Proteinen durch Endopeptidasen (Proteinasen) katalysiert, wogegen Peptidbindungen am Ende eines Peptid- oder Proteinmoleküls durch Exopeptidasen (früher Peptidasen) gespalten werden.

Dabei kann eine weitere Unterscheidung der Proteasen hinsichtlich ihrer im aktiven Zentrum für die Katalyse verantwortlichen Gruppen getroffen werden. So werden beispielsweise a) Serin-Proteasen, b) Cystein-Proteasen, c) Aspartat-Proteasen und d) Metallo-Proteasen voneinander unterschieden. Die Gruppe der Metallo-Proteasen (Metallo-Peptidasen) weisen beispielsweise in ihrem aktiven Zentrum Metallionen auf, die an dem katalytischen Mechanismus der Proteolyse beteiligt sind. Diese Metallionen, insbesondere divalente Metallkationen wie Magnesium, Zink, Calcium, Eisen u.a., werden auch als Coenzym betrachtet. Gemäß dem oben beschriebenen Unterscheidungsmerkmal gibt es weiterhin a) Metallo-Endopeptidasen (Metallo-Proteinasen) und b) Metallo-Exopeptidasen.

Matrix-Metallo-Proteasen (MMP) - auch Matrixine genannt - gehören zu einer Familie von Proteasen, die durch strukturelle Homologien definiert worden sind. Ihre enzymatische Aktivität ist abhängig von Metallionen im aktiven Zentrum. Metallo-Proteasen wurden zuerst durch ihre Rolle beim Gewebe-Umbau identifiziert, insbesondere durch den Abbau der extrazellulären Matrix. Systematisch gehört diese Gruppe der Proteasen zu den Metallo-Endopeptidasen (Metallo-Proteinasen). Zu den Matrix-Metallo-Proteinasen gehören unter anderem die Collagenasen, Gelatinasen, Stromeolysine, Matrilysin u.a.. Eine Übersicht und Einteilung ist in der Fachliteratur bei Parks, Matrix Metalloproteinases, Biology of Extracellular Matrix Series, Ed. Mecham, R. P., Academic Press, Inc., San Diego, Calif. (1998) und in der unten stehenden Tabelle 1 zu finden. In dieser Tabelle 1 sind Synonyme und gegebenenfalls, die Nummerierung der Enzyme gemäß der Enzym Commision (EC) angegeben. Die Matrix-Metallo-Proteinasen werden als inaktive Vorstufen synthetisiert und sezerniert. Durch komplexe, derzeit noch nicht im Detail endgültig geklärte Mechanismen werden sie in die aktive Form überführt. Alle Metallo-Proteasen können durch Ethylendiamintetraessigsäure (EDTA) gehemmt werden.

Serin-Proteasen weisen im aktiven Zentrum einen für die Katalyse essentiellen L-Serin-Rest auf, der durch Diisopropylfluorophosphat inhibiert werden kann. Auch diesen Proteasen wird in der Wundheilung eine entscheidende Funktion zugeschrieben. Zu der Gruppe der Serin-Proteasen behören beispielsweise Chromotrypsin, Elastase, Kallikrein, Plasmin, Trypsin, Thrombin u.a.. Die meisten bekannten Serin-Proteasen weisen neben dem L-Serin-Rest in ihrem aktiven Zentrum die Reste der Aminosäuren L-Histidin und L-Asparagin auf. Alle drei Aminosäurereste nehmen bei der Protolyse an einer Kaskade von Reaktionen teil, in der ein Proton des L-Serin-Restes auf das Substrat übertragen wird. Insoweit sich ein Serinabhängiger Mechanismus bei Proteinasen findet, spricht man auch von Serin-Proteinasen. Auch diese Serin-Proteasen werden durch Polyacrylatpartikel mit einer Partikelgröße x mit x ≤ 300 µm in ihrem Effekt auf die Wundheilung gehemmt.

**Tabelle 1: Matrix-Metallo-Proteasen (MMP)**

| Enzym | E.C.-Nr. | Pseudonym |
|---|---|---|
| MMP-1 | 3.4.24.7 | Collagenase-1, Fibroblastencollagenase |
| MMP-8 | 3.4.24.34 | Collagenase-2, Neutrophile Collagenase, |
| MMP-13 | | Collagenase-3, |
| MMP-18 | | Collagenase-4 |
| MMP-2 | 3.4.24.24 | Gelatinase-A |
| MMP-9 | 3.4.24.35 | Gelatinase-B |
| MMP-3 | 3.4.24.17 | Stromeolysin-1; Transin-1, Procollagenase |
| MMP-10 | 3.4.24.22 | Stromelysin-2; Transin-2 |
| MMP-11 | | Stromelysin-3 |
| MMP-7 | 3.4.24.33 | Matrilysin, PUMP-1 Protease |
| MMP-29 | | Matrilysin-2, Endometase |
| MMP-20 | | Enamelysin |
| MMP-28 | | Epilysin |
| MMP-12 | 3.4.24.65 | Makrophage Metallo-Elastase |
| MMP-19 | | RASI-1 |
| MMP-23 | | CA-MMP |
| MT1-MMP | 3.4.24.80 | Membran-Typ MMP-14 |
| MT2-MMP | | Membran-Typ MMP-15 |
| MT3-MMP | | Membran-Typ MMP-16 |
| MT4-MMP | | Membran-Typ MMP-17 |
| MT5-MMP | | MMP-24 |
| MT6-MMP | | MMP-25, Leukolysin |

Im Zusammenhang mit der vorliegenden Erfindung soll unter einer wässrigen Flüssigkeit Wasser, Salzlösungen, insbesondere physiologische Salzlösungen wie physiologische Kochsalzlösungen oder Ringerlösungen und Wundexsudat verstanden sein.

Die eingesetzten Polyacrylatpartikel mit einer Partikelgröße x mit 850 < x ≤ 1500 µm zeigen insbesondere bei Trägermaterialien, die ein von Fasermaterial verschiedenes Material beispielsweise ein Schaummaterial umfassen, eine besonders gute Leistung hinsichtlich ihrer Aufnahmekapazität und oder Abgabekapazität von wässrigen Lösungen. Somit muss die Wahl der Größenbereiche der Partikel zur Auf- und/ oder Abgabe von wässrigen Lösungen dem jeweiligen Trägermaterial angepasst werden.

Wie sich gezeigt hat, wird durch das Partikelgemisch aus Polyacrylatpartikeln mit einer Partikelgröße x mit 45 < x ≤ 300 µm und bevorzugt 150 < x ≤ 300 µm, das essentieller Bestandteil der Wundauflage ist, Proteasen, insbesondere Metallo-Proteasen wie beispielsweise Matrix-Metallo-Proteasen MMP-2 oder MMP-9 besonders gut gebunden oder kompartimentiert. Dies hat den Vorteil, dass durch die Wundauflage ein Überschuss an Proteasen, insbesondere an Metallo-Proteasen, auf ein für eine natürliche Wundheilung benötigte, reduzierte Menge gebracht werden kann. Diese gebundenen Metallo-Proteasen stehen nicht weiter als Inhibitoren der Wundheilung im Wundareal zur Verfügung. Durch einen Verbandwechsel können zudem diese Proteasen nachhaltig entfernt werden. Damit umfasst eine erfindungsgemäße Wundauflage ein Partikelgemisch aus Polyacrylatpartikeln mit einer Partikelgröße x mit x ≤ 300 µm als Proteasen-Inhibitor, wobei die Proteasen durch die Polyacrylatpartikel gebunden und/ oder kompartimentiert werden.

Diese Kompositionen können adaptiert an die Bedürfnisse einer Wunde werden. Sie können verstärkt Inhibitoren der Wundheilung binden oder falls diese in nicht nennenswert hohen Konzentrationen vorliegen, möglichst wenig in die normale Wundheilung durch das Abfangen von Wachstumsfaktoren und andere endogene, in der normalen Wundheilung physiologisch regulierte Mediatoren eingreifen.

Hierbei werden im Rahmen der vorliegenden Erfindung unter Polyacrylatpartikel diejenigen Partikel verstanden, die aus einem Polyacrylat gebildet werden. Dieses Polyacrylat ist ein synthetisches Polymer umfassend als Monomer (M1) Acrylsäure (2-Propensäure, CH₂=CH-CO₂H) und/ oder ein Salz hiervon, das einen Monomeranteil von mehr als 70 Gew.-% Acrylsäure und/ oder eines Salzes hiervon (bezogen auf das Gesamtgewicht des Polyacrylats) aufweist. Insbesondere weisen erfindungsgemäße Polyacrylate einen Monomeranteil von mehr als 80 Gew.-% Acrylsäure und/oder eines Salzes hiervon und ganz besonders bevorzugt mehr als 95 Gew.-% Acrylsäure und/oder eines Salzes hiervon bezogen auf das Gesamtgewicht des Polyacrylats auf. Damit umfasst eine erfindungsgemäße Wundauflage insbesondere ein Partikelgemisch aus Polyacrylatpartikeln, die einen Monomeranteil von mehr als 80 Gew.-% Acrylsäure und/oder eines Salzes hiervon und ganz besonders bevorzugt mehr als 95 Gew.-% Acrylsäure und/oder eines Salzes hiervon bezogen auf das Gesamtgewicht des Polyacrylats aufweisen, insbesondere als Mittel zur Hemmung von Proteasen in einer Wunde. Dabei kann das Polyacrylat als Homopolymer, Copolymer oder Blockpolymer vorliegen. Falls das Polyacrylat als Copolymer oder Blockpolymer vorliegt, so liegt in jedem Fall der Monomeranteil des Monomers M1 in dem Polymer bei mehr als 70 %, insbesondere bei mehr als 80 % und ganz besonders bevorzugt bei mehr als 95 % bezogen auf das Gesamtgewicht des Polyacrylats. In diesen copolymeren Polyacrylaten oder blockpolymeren Polyacrylaten können neben dem Monomer M1 als Comonomere M2 insbesondere α,β-ungesättigte Ether (Vinylether), α,β-ungesättigte Carbonsäuren oder α,β-ungesättigte Carbonsäureester (Vinylester) enthalten sein. Von den Comonomeren □M2 der α,β-ungesättigten Carbonsäuren werden besonders Methacrylsäure (2-Methylpropensäure), Ethacrylsäure (2-Ethylpropensäure), Crotonsäure (2-Butensäure), Sorbinsäure (*trans*-*trans*-2,4-Hexadiensäure), Maleinsäure (*cis*-2-Butendisäure) oder Fumarsäure (*trans*-2-Butendisäure) bevorzugt. In einer besonders bevorzugten Form der Erfindung kann jedoch auch vorgesehen sein, dass das Polyacrylat aus a) einem Homopolymer aus Acrylsäure und/oder b) einem Copolymer aus i) Acrylsäure und einem Salz der Acrylsäure, ii) aus Methacrylsäure und einem Salz der Methacrylsäure oder iii) aus Acrylsäure und Methacrylsäure und deren Salze besteht. Weiterhin kann jedoch auch vorgesehen sein, dass es sich bei dem Polyacrylat um eine Mischung von verschiedenen Polyacrylaten handelt.

Hierbei können insbesondere die α,β-ungesättigten Carbonsäuren als auch die Acrylsäure in neutralisierter Form als Salz, in nicht-neutralisierter Form als freie Säure oder in Mischungen hiervon vorliegen. Insbesondere haben sich Polyacrylate, die aus Acrylsäure und Salzen der Acrylsäure aufgebaut sind als besonders wirksam gezeigt. Dabei sind insbesondere Alkalimetall- oder Erdalkalimetallsalze hervorzuheben. Insbesondere zeigten sich Polyacrylate, die aus Homopolymeren und/oder aus Copolymeren bestehen, die als Monomere Acrylsäure und/ oder Natrium- oder Kaliumacrylat umfassen, als besonders wirksam.

Damit umfasst weiterhin bevorzugt ein erfindungsgemäßes Partikelgemisch Polyacrylatpartikeln, die aus Homopolymeren und/oder aus Copolymeren bestehen, die als Monomere Acrylsäure und/ oder Natrium- oder Kaliumacrylat umfassen, insbesondere als Mittel zur Hemmung von Proteasen.

Weiterhin hat sich in überraschender Weise gezeigt, dass die vorliegende Aufgabe in besonderes hervorzuhebender Weise durch Polyacrylate aus der Gruppe der vernetzten und/ oder quervernetzten und/ oder oberflächenvernetzten Polyacrylate gelöst wird. Diese Polyacrylate umfassen vorzugsweise a) ein Homopolymer, das aus den Monomeren M1 besteht und mittels eines Vernetzers vernetzt und/ oder quervernetzt ist, und/ oder b) ein Copolymer, das aus den Monomeren M1 und M3 besteht, wobei das Monomer M1 Acrylsäure und/ oder ein Salz hiervon ist und das Monomer M3 aus der Gruppe der Vernetzer gewählt wird. Das heißt, dass diese Polyacrylate ein mittels eines Vernetzers nachträglich vernetztes Polyacrylat und/ oder ein Polyacrylat umfassen, das aus Acrylsäure und/ oder einem Salz hiervon und einem Vernetzer copolymerisiert ist.

Damit umfasst weiterhin bevorzugt ein erfindungsgemäßes Partikelgemisch Polyacrylatpartikeln, die ein vernetztes und/ oder quervernetztes und/ oder oberflächenvernetztes Polyacrylat umfassen, insbesondere als Mittel zur Hemmung von Proteasen.

Insbesondere hat sich gezeigt, dass vernetze und/ oder quervernetzte Polyacrylate, die als Vernetzer Verbindungen V1 enthalten, die mindestens zwei ethylenisch ungesättigte Gruppen innerhalb eines Moleküls aufweisen, oder Verbindungen V2, die mindestens zwei funktionelle Gruppen aufweisen, die mit funktionellen Gruppen der Acrylsäure oder/ und eines Salzes hiervon in einer Kondensationsreaktion, in einer Additionsreaktion oder in einer Ringöffnungsreaktion reagieren können, oder Verbindungen V3, die mindestens eine ethylenisch ungesättigte Gruppe und mindestens eine funktionelle Gruppe, die mit funktionellen Gruppen der Acrylsäure und/ oder eines seiner Salze und/ oder den α,β-ungesättigte Comonomere in einer Kondensationsreaktion, in einer Additionsreaktion oder in einer Ringöffnungsreaktion reagieren kann, besonders wirksam sind. Dabei wird durch die Verbindungen V1 eine Vernetzung der Polymere durch die radikalische Polymerisation der ethylenisch ungesättigten Gruppen des Vernetzermoleküls mit den monoethylenisch ungesättigten Monomeren Acrylsäure und/ oder eines seiner Salze und/ oder einer der α,β-ungesättigte Comonomere erreicht, während bei den Verbindungen V2 eine Vernetzung der Polymere durch Kondensationsreaktion der funktionellen Gruppen mit den funktionellen Gruppen der Acrylsäure und/ oder eines seiner Salze oder einer α,β-ungesättigte der Comonomere erreicht wird. Bei den Verbindungen V3 erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren.

Bevorzugte Verbindungen V1 sind Polyacrylsäureester oder Polymethacrylsäureester, die beispielsweise durch die Umsetzung eines Polyols, wie beispielsweise Ethylenglykol (1,2-Ethandiol), Propylenglykol (1,2-Propandiol), Trimethylolpropan (2-Ethyl-2-hydroxymethyl-1,3-propandiol), 1,6-Hexandiol, Glycerin (1,2,3-Propantriol), Pentaerythrit (2,2-Bis(hydroxymethyl)propan-1,3-diol), Polyethylenglykol (HO-(CH₂-CH₂-O)ₙ-H mit n = 2 bis 20) mit n = 1 bis 20), Polypropylenglykol (HO-(CH(CH₃)-CH₂-O)ₙ-H mit n = 2 bis 20), eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Besonders bevorzugt kann es sich bei dem vernetzten Polyacrylaten um ein Polyacrylat handeln, das mittels einer Verbindung V1 vernetzt ist, die ein Di-, Tri- oder Tetraester der Polyacrylsäure oder Polymethacrylsäure ist, der durch die Umsetzung eines alkoxylierten Polyols, insbesondere eines ethoxylierten Polyols, insbesondere ethoxyliertes Ethylenglycol, ethoxyliertes Propylenglycol, ethoxyliertes Trimethylolpropan, ethoxyliertes 1,6-Hexandiol oder ethoxyliertes Glycerin, mit einer durchschnittlichen Anzahl an Ethylenoxideinheiten n pro Hydroxygruppe von n = 1 bis 10 mit Acrylsäure oder Methacrylsäure gewonnen werden. Als Verbindungen V1 sind des Weiteren Polyvinylverbindungen, Polyallylverbindungen, Polymethylallylverbindungen, Acrylsäureester oder Methacrylsäureester einer Monovinylverbindung, Acrylsäureester oder Methacrylsäureester einer Monoallylverbindung oder Monomethylallylverbindung, vorzugsweise der Monoallylverbindungen oder Monomethylallylverbindungen eines Polyols oder eines Aminoalkohols, bevorzugt. In diesem Zusammenhang wird auf DE 195 43 366, DE 195 43 368 verwiesen.

In jedem Fall weisen die Polyacrylatpartikel aus den aufgeführten Polyacrylaten in der Wundauflage in trockener Form die oben aufgezeigten Partikelgrößen auf. Diese Partikel können in der Wundauflage in trockener oder in bereits gequollener Form zum Einsatz gebracht werden. In gequollener Form liegen die Polyacrylatpartikel in gelförmigen Partikeln vor. Dabei können die Polyacrylatpartikel mit Wasser, physiologischer Kochsalz-Lösung oder Ringer-Lösung versetzt sein.

Insbesondere weist eine Wundauflage vor dem Gebrauch ein erfindungsgemäßes Partikelgemisch aus Polyacrylatpartikeln auf, das höchstens 10 Gew.-% Wasser (bezogen auf das Gesamtgewicht der Polyacrylatpartikel) umfasst, d.h., dass die Polyacrylatpartikel einen Feuchtigkeitsgehalt von höchstens 10 % aufweisen. Insbesondere ist dabei vorgesehen, dass Wundauflage ein Partikelgemisch umfasst, das höchstens 7 Gew.-%, vorzugsweise höchstens 5 Gew.-% Wasser und ganz besonders bevorzugt höchstens 4 Gew.-% Wasser (bezogen auf das Gesamtgewicht der Polyacrylatpartikel) umfasst.

Gemäß einem weiterführenden Gedanken kann ein erfindungsgemäßes Partikelgemisch in einer vielschichtigen Wundauflage bereitgestellt werden. Hierbei ist insbesondere vorgesehen, dass die Wundauflage neben einer ersten, das Partikelgemisch und das Trägermaterial für das Partikelgemisch umfassenden Lage eine zweite, weitere Lage zum Einsatz gebracht wird. Diese zweite Lage kann vielfältige Funktionen übernehmen. Hierbei ist insbesondere auch vorgesehen, dass die Wundauflage weiterhin eine das Partikelgemisch und das Trägermaterial für das Partikelgemisch von einer Wunde beabstandende Wundkontaktschicht oder weiterhin eine das Partikelgemisch und das Trägermaterial für das Partikelgemisch umschließende Umhüllung umfasst. Hierdurch ist insbesondere gewährleistet, dass im anwendungsbestimmten Zustand der Wundauflage keine Partikel in die Wunde gelangt. Somit weist diese Wundauflage umfassend das Partikelgemisch, das Trägermaterial und die Umhüllung eine erste, das Partikelgemisch und das Trägermaterial umfassende Lage, sowie eine zweite und eine dritte aus der Umhüllung gebildete Lage auf. Die Umhüllung oder die Wundkontaktschicht kann hierbei insbesondere aus einem Nonwoven, einem Gestrick, einem Gewirk oder einem Gewebe gebildet werden, wobei diese Wundkontaktschicht oder diese Umhüllung weiterhin bevorzugt keine Partikel aufweisen. Besonders bevorzugt sind hierbei Nonwoven, da diese Materialien sehr dicht sind und keine Partikel in eine Wunde gelangen lassen. Nicht-wundverklebende Wundauflagen unterstützen in besonderem Maße eine Wundheilung in der Granulationsphase der Wundheilung, da sie einerseits durch den Gehalt an Partikeln mit einer Partikelgröße x mit x ≤ 300 µm Proteasen, insbesondere durch Kompartimentierung oder Bindung in ihrer Aktivität hemmen, und andererseits neu gewachsenes Gewebe bei einem Verbandwechsel nicht beschädigen. Diese nichtwundverklebenden Wundauflagen umfassen als Wundkontaktschicht insbesondere ein nichtwundverklebendes Gestrick, Gewirk, Gewebe oder Nonwoven, das weiterhin bevorzugt aus einem hydrophoben Fasermaterial besteht. Insbesondere kann die Wundkontaktschicht ein Gestrick, Gewirk oder Gewebe aus einem hydrophoben Polyethylen-, Polypropylen-, Polyester- oder Viskose-Polymermaterial bestehen. Durch die Ausgestaltung als Gestrick, Gewirk oder Gewebe kann die Wundkontaktschicht in einer oder mehreren Richtungen gedehnt oder verformt werden, ohne dass sie sich von selbst wieder zusammenzieht oder ausrichtet. Die Oberfläche einer derartigen Wundkontaktschicht gleicht sich zudem passgenau an die Oberfläche der zu behandelnden Haut oder Wunde an. Alternativ kann die Wundauflage auch ein Partikelgemisch aus Polyacrylatpartikeln und ein Trägermaterial für das Partikelgemisch umfassen, wobei das Trägermaterial ein hydrophiles Fasermaterial umfasst und wobei insbesondere die Polyacrylatpartikel ein vernetzes und/ oder quervernetztes Polyacrylat umfassen. Hierbei können als hydrophiles Fasermaterial insbesondere wasserunlösliche Fasern aus Cellulose, insbesondere weitgehend delignifizierte technische Zellstofffasern, insbesondere Holzzellstofffasern, insbesondere einer Faserlänge von < 5 mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten. Es kann auch vorgesehen sein eine Fasermischung aus Cellulose-, regenerierter Cellulose, Carboxymethylcellulose-, Carboxyethylcellulose-, Hydroxymethylcellulose- oder Hydroxyethylcellulose-Fasern und Fasern aus Polyethylen, Polypropylen oder Polyester vorzusehen. In einer ganz besonders bevorzugten Ausführungsform umfasst die Wundauflage ein Partikelgemisch aus Polyacrylatpartikeln der angegebenen Zusammensetzung und eine Mischung aus Cellulosefasern, Polypropylenfasern als Trägermaterial für das Partikelgemisch.

Damit umfasst eine Wundauflage ein Partikelgemisch der oben aufgezeigten Zusammensetzung und ein Trägermaterial für das Partikelgemisch, das als erste Fasern Stapelfasern aus synthetischen und/ oder natürlichen Polymeren enthält. Insbesondere umfasst diese Wundauflage erste hydrophile Stapelfasern. Diese Fasern können in einem sogenannten Air-laid-Verfahren zusammen mit dem Partikelgemisch zu einer Schicht verarbeitet sein.

Je nach geplantem Einsatz kann eine Wundauflage unterschiedliche Mengen an Polyacrylatpartikeln und Trägermaterial für die Polyacrylatpartikel aufweisen. Dabei umfasst die Wundauflage mindestens 10 Gew.-% Polyacrylatpartikel (bezogen auf das Trägermaterial), wobei die Polyacrylatpartikel die im Zusammenhang mit der vorliegenden Erfindung aufgeführten Zusammensetzung aufweisen. Besonders bevorzugt sind jedoch Wundauflagen, die mindestens 20 Gew.-%, insbesondere mindestens 25 Gew.-% und ganz besonders bevorzugt mindestens 30 Gew.-% Polyacrylatpartikel (bezogen das Trägermaterial) umfassen. Um die Wundauflage in ihren Leistungseigenschaften hinsichtlich der Hemmung der Aktivitäten von Proteasen und/ oder der Auf- und Abgabe von wässrigen Flüssigkeiten nicht einzuschränken, sollte jedoch gewährleistet sein, dass der Gehalt an Polyacrylatpartikeln in Bezug auf das Trägermaterial insbesondere nicht mehr als 80 Gew.-% und insbesondere nicht mehr als 75 Gew.-% beträgt. In einer weiteren alternativen Ausführung ist vorgesehen, dass eine erfindungsgemäße vielschichtige Wundauflage eine erste partikelhaltige Schicht A und mindestens eine zweite partikelhaltige Schicht B umfasst. Hierbei umfasst die erste Schicht A Partikel einer ersten Größe und die zweite Schicht B Partikel einer zweiten, von der ersten Größe verschiedenen Größe. Je nach geplantem Einsatz kann auch eine vielschichtige Wundauflage unterschiedliche Mengen an Polyacrylatpartikeln und Trägermaterial für die Polyacrylatpartikel aufweisen. Falls vorgesehen ist, dass die Partikel in unterschiedlichen Schichten angeordnet werden, soll die Partikelmenge mindestens 10 Gew.-% bezogen auf das Trägermaterial aller partikelhaltigen Lagen betragen. Insbesondere ist auch hierbei vorgesehen, dass Wundauftage mit mindestens 20 Gew.-%, insbesondere mindestens 25 Gew.-% und insbesondere mindestens 30 Gew.-% Polyacrylatpartikel (bezogen auf das Trägermaterial aller partikelhaltigen Schichten) besonders bevorzugt sind. Um die Wundauflage in ihren Leistungseigenschaften hinsichtlich der Hemmung der Aktivitäten von Proteasen und/ oder der Auf- und Abgabe von wässrigen Flüssigkeiten nicht einzuschränken, sollte jedoch gewährleistet sein, dass der Gehalt an Polyacrylatpartikeln in Bezug auf das Trägermaterial insbesondere nicht mehr als 80 Gew.-% und insbesondere nicht mehr als 75 Gew.-% (bezogen auf das Trägermaterial aller partikelhaltigen Schichten) beträgt. Die vorliegende Erfindung betrifft die auch die Verwendung eines Partikelgemischs insbesondere in einer Wundauflage umfassend Polyacrylatpartikel unterschiedlicher Größe zur Hemmung oder Inhibition von Proteasen in einer Wunde, wobei das Partikelgemisch
- a) 20 bis 98 Gew.-% Partikel mit einer Partikelgröße x mit 45 ≤ x ≤ 300 µm und
- b) 2 bis 80 Gew.-% Partikel mit einer Partikelgröße x mit x > 300 µm umfasst.
In einer weiteren vorteilhaften Verwendung werden die Polyacrylatpartikel mit einer Partikelgröße x mit 45 ≤ x ≤ 300 zur Herstellung eines Mittels zur Hemmung oder Inhibition durch Kompartimentierung von Proteasen, insbesondere Metallo-Proteasen und ganz besonders bevorzugt von Matrix-Metallo-Proteasen in chronischen Wunden eingesetzt. Diese Polyacrylate können zudem vorzugsweise zur Inhibition von Proteasen, insbesondere Metallo-Proteasen und ganz besonders bevorzugt von Matrix-Metallo-Proteasen zum Gewebeaufbau und/ oder zur Regulation des Gewebeaufbaus in chronischen Wunden eingesetzt werden.

Chronische Wunden können definiert werden als Wunden, deren Heilungsverlauf in einem oder allen Stadien der Wundheilung von der normalen Wundheilung abweichen. So kann aus akuten, normal heilenden Wunden z.B. durch eine Wundinfektion eine chronische Wunde entstehen, die durch eine verzögerte Heilungsgeschwindigkeit gekennzeichnet ist. Der Übergang von einer akuten zu einer chronischen Wunde kann dabei in jedem Stadium der Wundheilung erfolgen. Klinisch werden chronische Wunden definiert als Wunden, deren Heilung mehr als 6-8 Wochen benötigen, wobei diese Definition nicht alle Krankheitsbilder korrekt abdeckt. Es handelt sich bei chronischen Wunden mehr um eine Diagnose, die sich auf die klinische Erfahrung des medizinischen Personals stützt.

Chronische Wunden entstehen insbesondere aufgrund einer mechanischen Belastung (Dekubitus, Druck-Ulzera, Druckgeschwür), einer venösen Insuffizienz (Ulcus cruris venosum, venöse Ulzera), einer artheriosklerotischen Gefäßveränderung (Ulcus cruris arteriosum, arterielle Ulzera), einer neuropathischen Veränderungen (diabetisches Fußsyndrom, neuropathische Ulzera), aber auch in Folge von Autoimmunerkrankungen, von Tumoren (exulzierende Tumore) oder Strahlenschäden bei der Tumortherapie.

Der Dekubitus ist definiert als durch äußere (längerfristige) Druckeinwirkung mit Kompression von Gefäßen und lokaler Ischämie hervorgerufene trophische Störung von Geweben (v. a. Haut und Unterhautgewebe) mit Nekrose, Mazeration, evtl. Infektion. Dekubitalulcera entstehen vor allem bei Bettlägerigkeit, insbesondere an Körperstellen, an denen die Haut dem Knochen unmittelbar anliegt, aber auch z.B. unter schlecht sitzenden Prothesen und zu engen Gipsverbänden.

Der Dekubitus wird in folgende Stadien eingeteilt. Hierbei sind als chronische Wunden insbesondere Dekubita der Stufe II, Stufe III und Stufe IV bekannt:
- Dekubitus - Stufe I: Hierbei handelt es sich um eine persistierende, umschriebene Hautrötung, die auch bei Entlastung bestehen bleibt. Die Rötung ist scharf umgrenzt und kann verhärten oder überwärmt sein. Die Haut ist noch intakt.
- Dekubitus - Stufe II: In dieser Phase kommt es zu Blasenbildung und Hautabschürfung und damit zu Teilverlust der Haut. Die Epidermis bis hin zu Anteilen der Dermis ist geschädigt. In dieser Phase liegt eine oberflächige Wunde oder flaches Geschwür vor.
- Dekubitus - Stufe III: In diesem fortgeschrittenen Stadium ist bereits ein Verlust aller Hautschichten zu beobachten. Darüber hinaus sind eine Schädigung der subkutanen Gewebe und eventuell Nekrosen zu beobachten, die bis auf das darunter liegende Muskelgewebe reichen können. Erfahrungsgemäß muss es erst zu einer Abgrenzung des nekrotischen Gewebes kommen bis das ganze Ausmaß des Gewebeschadens erkennbar wird. Der Dekubitus III zeigt sich klinisch als offenes, tiefes Geschwür.
- Dekubitus - Stufe IV: In diesem äußerst kritischen Stadium ist ein Verlust aller Hautschichten mit ausgedehnter Zerstörung, Gewebsnekrose oder Schädigung von Muskeln, Knochen oder unterstützenden Strukturen (Sehnen, Gelenkkapseln) zu verzeichnen. Der Dekubitus IV zeigt sich klinisch als großflächiges, offenes und tiefes Geschwür.

Damit betrifft eine Weiterbildung der vorliegenden Erfindung auch die Verwendung eines Partikelgemischs aus Polyacrylatpartikeln, das jede oben aufgeführte Zusammensetzung aufweist, als Mittel zur Hemmung von Proteasen, insbesondere Metallo-Proteasen, zur Behandlung von und zur Herstellung eines Mittels zur Behandlung von Dekubitus, Ulcus cruris venosum, Ulcus cruris arteriosum oder diabetisches Fußsyndrom. Insbesondere betrifft die vorliegende Erfindung auch die Verwendung eines Partikelgemischs aus Polyacrylatpartikeln, das
a) 20 bis 98 Gew.-% Partikel mit einer Partikelgröße x mit 45 ≤ x ≤ 300 µm und
b) 2 bis 80 Gew.-% Partikel mit einer Partikelgröße x mit x > 300 µm umfasst
als Mittel zur Hemmung von Proteasen, insbesondere Metallo-Proteasen, zur Behandlung von und zur Herstellung eines Mittels zur Behandlung von Dekubitus, Ulcus cruris venosum, Ulcus cruris arteriosum oder diabetisches Fußsyndrom. Dabei wird der Gewebeaufbau durch die Ausschaltung der die Wundheilung störenden Mechanismen gefördert, was sich klinisch in einer Besserung des Wundzustandes sowie der Wundverkleinerung bemerkbar macht.

In einer weiteren vorteilhaften Verwendung werden die Polyacrylatpartikel zur Hemmung durch Kompartimentierung von Proteasen, insbesondere Metallo-Proteasen und zur Herstellung eines Mittels zur Hemmung durch Kompartimentierung von Proteasen, insbesondere Metallo-Proteasen bei Dekubitus, Ulcus cruris venosum, Ulcus cruris arteriosum oder diabetisches Fußsyndrom eingesetzt. Diese Polyacrylatpartikel können zudem vorzugsweise zur Inhibition von Metallo-Proteasen oder Matrix-Metallo-Proteasen zum Gewebeaufbau und/ oder zur Regulation des Gewebeaufbaus bei diesen Krankheiten eingesetzt werden.

Nachstehend wird die Erfindung unter Bezugnahme auf Zeichnungen erläutert. In den Zeichnungen zeigt:
- Figur 1 bis 3:
- Drei verschiedene Wundauflagen mit Polyacrylatpartikeln jeweils im Querschnitt
- Figur 4:
- Die unterschiedliche Bindung von Metallo-Proteasen aus dem Exsudat chronischer Wunden in Abhängigkeit von der Partikelgrösse der Polyacrylatpartikel

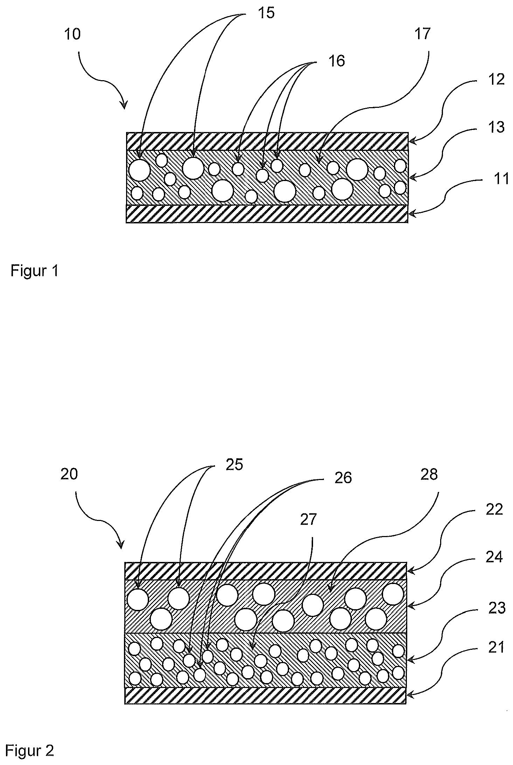

Mit Figur 1 ist eine Wundauflage (10) in einer ersten erfindungsgemäßen Ausführungsform gezeigt. Die Wundauflage weist eine erste, in bestimmungsgemäßer Anwendung auf einer Wunde aufzulegende Abstandschicht (11) und eine erste Deckschicht (12) auf. Zwischen diesen beiden Schichten befindet sich eine weitere Schicht (13), die ein Trägermaterial (17) und ein erfindungsgemäßes Partikelgemisch aus Polyacrylatpartikeln (15, 16) umfasst. Das Partikelgemisch enthält zu gleichen Anteilen Polyacrylatpartikel (16) einer ersten Partikelgröße x mit 45 ≤ x ≤ 300 µm und Polyacrylatpartikel (15) einer zweiten Partikelgröße x mit 600 ≤ x ≤ 850 µm. Damit enthält das Partikelgemisch 50 Gew.-% Polyacrylatpartikel (16) einer ersten Partikelfraktion mit einer ersten Partikelgröße x mit 45 ≤ x ≤ 300 µm und 50 Gew.-% Polyacrylatpartikel (15) einer zweiten Partikelfraktion mit einer zweiten Partikelgröße x mit 300 〈 x ≤ 850 µm (bezogen auf das Gesamtgewicht an Partikeln). Das Trägermaterial (17) für das Partikelgemisch ist ein Fasermaterial aus hydrophilen Stapelfasern, die 94 Gew.-% hydrophilen Cellulose-Fasern und 6 Gew.-% Polypropylenfasern. Das Trägermaterial (17) und die Polyacrylatpartikel (15, 16) sind in einem Air-Laid-Verfahren zu einer Lage verarbeitet, wobei die Lage ein Flächengewicht von 360 g/ m2 aufweist. Der Anteil an Fasermaterial in der Lage beträgt 198 g/ m2, wobei der Anteil an Polyacrylatpartikeln (15, 16) 162 g/m2 beträgt. Damit entspricht der Anteil an Polyacrylatpartikeln (15, 16) 45 Gew.-% bezogen auf das Trägermaterial (17). Die erste Abstandsschicht (11) und die erste Deckschicht (13) sind aus einem Nonwoven aus einem hydrophoben Fasermaterial aus Polypropylenfasern gefertigt. Somit weist diese Wundauflage eine erste Abstandsschicht als Wundkontaktschicht auf, die keine Wundverklebung zeigt.

Mit Figur 2 ist eine alternative nicht erfindungsgemäße Wundauflage (20) gezeigt. Diese Wundauflage weist eine Wundkontaktschicht (21) aus einem hydrophoben Nonwoven aus Polyesterfasern auf. Über dieser Wundkontaktschicht (21) sind eine erste polyacrylatpartikelhaltige Schicht (23), eine zweite polyacrylatpartikelhaltige Schicht (24) und eine Abdeckschicht (22) angeordnet. Hierbei weisen die Polyacrylatpartikel (26) der ersten partikelhaltigen Schicht (23) eine Partikelgröße x mit 150 ≤ x ≤ 300 µm und die Polyacrylatpartikel (25) der zweiten partikelhaltigen Schicht (24) eine Partikelgröße x mit 600 ≤ x ≤ 1500 µm auf. In der ersten partikelhaltigen Schicht (23) sind 126 g/ m2 Polyacrylatpartikel mit einer Partikelgröße x mit 150 ≤ x ≤ 300 µm (26) und 198 g/ m2 hydrophile Stapelfasern enthalten. In der zweiten partikelhaltigen Schicht (24) sind 42 g/ m2 Polyacrylatpartikel mit einer Partikelgröße x mit 600 ≤ x ≤ 1500 µm (25) und 63 g/ m2 hydrophile Stapelfasern enthalten. Damit weist die erste Schicht (23) ein Flächengewicht von 315 g/ m2 und die zweite Schicht (24) ein Flächengewicht von 104 g/ m2 auf, wobei der Partikelanteil bezogen auf das Trägermaterial der partikelhaltigen Schichten (23, 24) 40 Gew.-% beträgt und wobei 75 Gew.-% Polyacrylatpartikel mit einer Partikelgröße x mit mit 150 ≤ x ≤ 300 µm (bezogen auf die Gesamtmenge an Polyacrylatpartikel) in der ersten Lage (23) und 25 Gew.-% Polyacrylatpartikel mit einer Partikelgröße x mit 600 ≤ x ≤ 1500 µm (bezogen auf die Gesamtmenge an Polyacrylatpartikeln) in der zweiten Lage (24) enthalten sind. Das Trägermaterial (27, 28) der ersten und der zweiten partikelhaltigen Schicht (23, 24) ist ein hydrophiles Fasermaterial (hydrophile Stapelfasern) aus 90 % Cellulosefasern und 10 Gew.-Polypropylenfasern, wobei sowohl die erste als auch die zweite partikelhaltige Schicht in einem Air-laid-Verfahren hergestellt ist. Im vorliegenden Fall wurden die beiden Lagen in einem In-Line-Prozess in aufeinander folgenden Schritten gefertigt. Das Gesamtgewicht der beiden partikelhaltigen Lagen (23, 24) beträgt 420 g/ m2. Die Abdeckschicht (22) besteht aus einem wasserdampfdurchlässigen Polyurethanfilm mit einer Dicke von 60 µm.

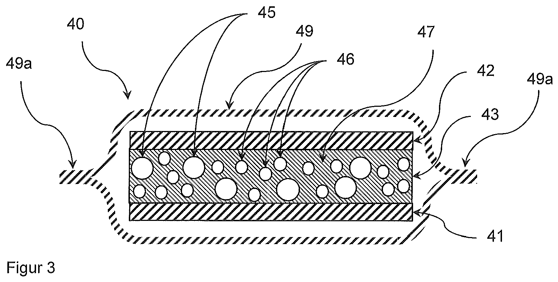

Mit Figur 3 ist eine dritte erfindungsgemäße alternative Wundauflage (40) gezeigt. Diese Wundauflage weist einen ähnlichen Aufbau wie die mit Figur 1 gezeigte Wundauflage auf, wobei die Wundauflage eine partikelhaltige Schicht (43) umfasst, die auf beiden Seiten mit einem Tissue (31, 32) abgedeckt ist. Die Tissueschichten bestehen aus 100 % Cellulosefasern und weisen ein Flächengewicht von 18 g/ m2 auf. Die partikelhaltige Schicht (43) besteht aus einem Trägermaterial (47) und einem Partikelgemisch aus Polyacrylatpartikeln (45, 46). Das Partikelgemisch enthält 65 g/ m2 Polyacrylatpartikel (46) einer ersten Partikelgröße x mit 150 ≤ x ≤ 300 µm und 97 g/ m2 Polyacrylatpartikel (45) einer zweiten Partikelgröße x mit 600 ≤ x ≤ 850 µm. Damit enthält das Partikelgemisch 40 Gew.-% Polyacrylatpartikel (46) einer ersten Partikelfraktion mit einer ersten Partikelgröße x mit 150 ≤ x ≤ 300 µm und 60 Gew.-% Polyacrylatpartikel (45) einer zweiten Partikelfraktion mit einer zweiten Partikelgröße x mit 600 〈 x ≤ 850 µm (bezogen auf das Gesamtgewicht an Partikeln). Die einzelnen Partikelfraktionen wurden aus Polyacrylatpartikeln (Favor PAC 300 - Stockhausen, Krefeld) ausgesiebt. Diese Partikel bestehen aus einem vernetzten Natriumpolyacrylat und weisen einen Wassergehalt von 3,0 Gew.-% Wasser bezogen auf die Polyacrylatpartikel auf. Das Trägermaterial (47) für das Partikelgemisch ist ein Fasermaterial aus hydrophilen Stapelfasern, die 94 Gew.-% hydrophilen Cellulose-Fasern und 6 Gew.-% Polypropylenfasern. Das Trägermaterial (47) und die Polyacrylatpartikel (45, 46) sind in einem Air-Laid-Verfahren zu einer Schicht verarbeitet, wobei die Schicht ein Flächengewicht von 360 g/ m2 aufweist. Der Anteil an Fasermaterial in der Lage beträgt 198 g/ m2, wobei der Anteil an Polyacrylatpartikeln (45, 46) 162 g/ m2 beträgt. Damit entspricht der Anteil an Polyacrylatpartikeln (45, 46) 45 Gew.-% bezogen auf das Trägermaterial (47). Die Wundauflage ist weiterhin von einer äußeren Umhüllung (39) aus einem hydrophoben Gestrick aus Polyethylen umgeben, das im entspannten Zustand eine Dicke von 0,8 mm aufweist. Dieses Gestrick verhindert die Wundverklebung und weist eine gute Modellierfähigkeit auf, so dass sich die gesamte Wundauflage dem Wundgrund anpassen kann. An den umlaufenden Rändern (39a, 39b) ist die Umhüllung allseitig thermoversiegelt.

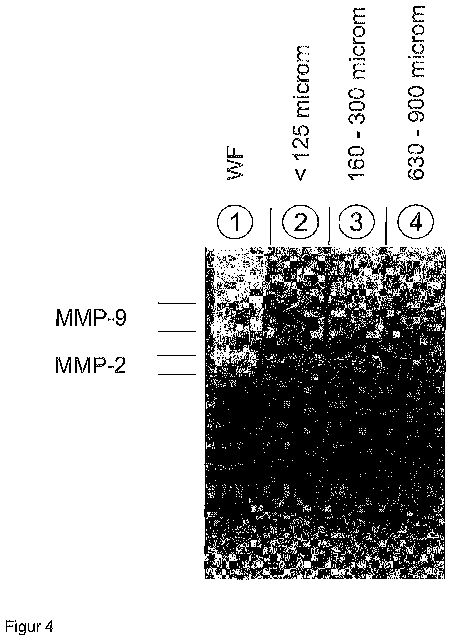

Mit Figur 4 ist die unterschiedliche Bindung von Metallo-Proteasen an Polyacrylatpartikel dargestellt.

Im folgenden Beispiel wurden Polyacrylatpartikel (vernetztes Polyacrylat; Favor PAC 300, Fa. Stockhausen, Krefeld - Deutschland) durch Siebung in verschiedene Größenbereiche aufgetrennt. So wurden Fraktionen mit Partikelgrößen < 125 µm, zwischen 160 und 300 µm und zwischen 630 und 900 µm untersucht. Die Partikel wurden mit Ringerlösung (8,6 g NaCl, 0,3 g KCl, 0,33 g CaCl_{2 *} 2 H₂O ad 1000 ml Wasser) voraktiviert; hierbei wurden 0,2 g Polyacrylatpartikel mit 36,94 ml Ringerlösung für 24 Stunden in einem abgeschlossenen Gefäß stehen gelassen wird und ein eventueller Überschuss an Ringerlösung verworfen. 100 mg der voraktivierten Polyacrylatpartikel wurden mit 20 µg Wundexsudatprotein in 50 µl für 2 Stunden unter konstantem Schütteln bei Raumtemperatur inkubiert. Das Wundexsudatprotein wurde von Patienten mit Ulcus cruris gewonnen, der Proteingehalt mit dem Biorad Proteinassay (Bio-Rad DC Protein Assay Kit II, Katalognummer 500-0112), bestimmt und mit Ringerlösung auf 20 µg/ 50 µl eingestellt. Der Überstand wurde nach der Inkubation abgenommen und der so behandelte Polyacrylat-Superabsorber wurde 3x mit einem Überschuss (5 w/w) Waschpuffer (10 g Bovine Serum Albumin (Sigma A-2153), 8,6 g NaCl, 0,3 g KCl, 0,33 g CaCl_{2 *} 2 H₂O ad 1000 ml Wasser) gewaschen. Das so behandelte Polyacrylat wurde mit 1 Volumen (v/w) 2x SDS-Gel Probenpuffer versetzt, bei 95 °C für 10 Minuten im Wasserbad aufgekocht und anschließend bei -20 °C gelagert oder direkt der Gelatine-Zymographie unterzogen. Dazu wurde ein Aliquot der Wundflüssigkeit, des mit Polyacrylat behandelten Überstandes and der Polyacrylat gebundenen Proteine auf ein Gelatine enthaltendes SDS Gel aufgetragen und elektrophoretisch aufgetrennt.

Diese Technik ist äußerst sensitiv und basiert auf einer Auftrennung der Proteasen in einem SDS-Gel, welches gleichzeitig ein Proteasesubstrat (Gelatine) enthält.

Das Gel setzt sich zusammen aus (alle Chemikalien Fa. Sigma-Aldrich Chemie GmbH, 89555 Steinheim - Deutschland):
- 3,3 ml Gelatinelösung (Gelatine, porcine skin (CAS-Nr. 9000-70-8), 3 mg/ ml H₂O),
- 0,85 ml destilliertes H₂O,
- 2,5 ml 1,5M Tris (Tris(hydroxymethyl)aminomethan) /
   HCl/ 0,4% SDS-Lösung (**S**odium**d**odecyl**s**ulfat-Lösung), pH 8,8,
- 3,35 ml 30% Acrylamid/ 0,8% Bisacrylamid Lösung,
- 50 µl (10%, w/v) Ammoniumpersulfat und
- 5 µl TEMED (*N, N, N', N*'-Tetramethylendiamin);
   das Obergel entspricht
- 3,075 ml destilliertes H₂O,
- 0,625 ml 0,5M Tris (Tris(hydroxymethyl)aminomethan) /
   HCl/ 0,4% SDS-Lösung (**S**odium**d**odecyl**s**ulfat-Lösung), pH 6.8,
- 0,75 ml 30% Acrylamid/ 0,8% Bisacrylamid Lösung,
- 50 µl (10%, w/v) Ammoniumpersulfat und
- 5 µl TEMED (*N, N, N', N*'-Tetramethylendiamin)

Die Gele können als so genannte Minigele oder in anderen Apparaturen als konventionelle Zymogramme zur Auftrennung des Proteingemisches verwendet werden. Dem Fachmann sind dies Apparaturen und Methoden bekannt.

Der Probenauftragspuffer kann ein beliebiger Proteinauftragspuffer sein, der keine reduzierenden Substanzen erhalten darf. Nach der Auftrennung werden die Proteasen renaturiert (waschen für 2x15 Min. in 2,5% Triton-X-100 in H₂O, dann waschen für 2x15 Min. in 50 mM Tris/ HCl pH 7,4; 5 mM CaCl₂) und für 24-48 Std. in dem letzten Waschpuffer inkubiert. Die renaturierten Metallo-Proteasen bauen das Gelatinesubstrat in ihrer unmittelbaren Nachbarschaft ab. Färbt man diese Zymogramme mit einem Proteinfarbstoff (Coomassie CAS-Nr. 6104-59-2, Fa. Sigma-Aldrich Chemie GmbH, 89555 Steinheim - Deutschland), gefolgt von der Entfärbung nach Standardprotokollen, stellt sich die nicht abgebaute Gelatine homogen blau im Gel dar (in der Abbildung Figur 5 grau/ schwarz dargestellt). Nur an Stellen an denen sich proteolytische Aktivität befindet und die Gelatine abgebaut wurde, imponieren klare, durchsichtige Banden, sogenannte "Fressbanden" (in der Abbildung Figur 5 weiß auf grauem/ schwarzen Grund dargestellt). An Hand des typischen Musters und des durch einen Proteinmarker ermittelten Molekulargewichtbereichs (hier nicht dargestellt) konnte die Art des Enzyms bestimmt werden. Die hier beschriebene Methode und Variationen davon (z.B. Herron et al., J Biol Chem. 1986 261:2814-8) sind dem Fachmann geläufig. Eine semiquantitative Auswertung erfolgte durch die Bildanalyse Software ImageJ (Image Processing and Analysis in Java, http://rsb.info.nih.gov/ij/index.html, last accessed 19.11.2007) durch densitometrische Auswertung der Bandenintensitäten gemäß der Softwareanleitung (http://rsb.info,nih.gov/ij/docs/index.html, last accessed 19.11.2007).

In diesen Experimenten war eine deutliche Bindung der Metallo-Proteasen an die verschiedenen Polyacrylatpartikel-Fraktionen in Abhängigkeit zur Partikelgröße festzustellen. Die semiquantitative Auswertung ist in Tabelle 2 dargestellt.

In der ersten Bahn (WF) sind 20 µl des verdünnten Ausgangswundexsudats aufgetragen. In der 2., 3. und 4. Bahn wurde das gebundene Wundexsudat in 25 µl Probenauftragspuffer durch Erhitzung abgelöst, wobei nur ein sehr geringer Teil auf den Polyacrylatpartikel verbleibt. Die so gewonnenen Proteine wurden auf die Gelatinezymogramme aufgetragen und entwickelt. Dabei wurde die Bandenintensitäten der 1. Bahn (Wundexudat) als Referenz mit 100 festgesetzt. Die Intensitäten der Bahnen 2, 3 und 4 sind als Prozentangaben auf die Referenz angegeben.

**Tabelle 2**

| | Bahn 1 | Bahn 2 | Bahn 3 | Bahn 4 |
|---|---|---|---|---|
| | WF | < 125 µm | 160 < x < 300 µm | 630 < x < 900 µm |
| MMP-9 | 100% (±0%) | 61.2% (±7.3%) | 79.3% (±5.7%) | 10.9% (±2.8%) |
| MMP-2 | 100% (±0%) | 44.5% (±4.7%) | 34.2% (±2.0%) | 7.2% (±1.0%) |

Es zeigt sich klar, dass in den Bahnen 2 (Polyacrylatpartikel mit einem Partikeldurchmesser von weniger als 125 µm) und Bahn 3 (Polyacrylatpartikel mit einem Partikeldurchmesser von 160 bis 300 µm) deutlich stärkere Signale für MMP-9 und MMP-2 Banden aufweisen. Durch die Waschschritte nach der Inkubation der Polyacralatpartikel mit dem Wundexsudat muss von einer starken Bindung ausgegangen werden. So binden Polyacrylatpartikel mit einem Partikeldurchmesser x mit 160 < x < 300 im Vergleich zu Polyacrylatpartikeln mit einem Partikeldurchmesser x mit 630 < x < 900 die Metalloprotease MMP-9 ca. siebenmal besser. Dieselbe Partikelfraktion bindet auch im Vergleich zu Polyacrylatpartikeln mit einem Partikeldurchmesser x mit 630 < x < 900 die Metallo-Protease MMP-2 ca. viermal besser.

Die Inhibierung der Matrix-Metallo-Proteasen mit der Folge der Heilung von chronischen Wunden konnte in klinischen Untersuchungen gezeigt werden. In dieser Untersuchung wurden drei repräsentative Patienten, die an einem offenen Unterschekelgeschwür (Ulcus cruris) leiden, mit einer Wundauflage behandelt, die den mit Figur 4 beschriebenen Aufbau aufweist. Dabei wurde die Partikelfraktion 40 Gew.-% Polyacrylatpartikel mit einer Fraktion von 150 < x ≤ 300 µm gewählt, um bei diesen Patienten überschießende Proteasemengen zu hemmen und die Wundheilung zu induzieren. Besonderes Augenmerk verdient dabei die Initiation des Granulationsgewebes, damit neues Bindegewebe aufgebaut werden kann. Der Verbandwechsel erfolgte alle 24 Stunden oder jeden 2. Tag. Die Ergebnisse sind in der nachfolgenden Tabelle zusammengefasst.

**Tabelle 3**

| | Start | | | | | Ende | | | |
|---|---|---|---|---|---|---|---|---|---|
| | Tag | Beläge | Granulationsgewebe | Wundgröße | | Tag | Beläge | Granulationsgewebe | Wundgröße |
| Patient 1 | 1 | 4 | 1 | 25 cm² | | 34 | 1 | 4 | 20 cm² |
| Patient 2 | 1 | 4 | 1 | 20 cm² | | 12 | 1 | 4 | 19 cm² |
| Patient 3 | 1 | 3 | 2 | 12 cm² | | 21 | 0 | 5 | 5 cm² |

Der Wundstatus der Ulzera wurde semiquantitativ in einer Skala von 0 bis 5 klinisch erfasst.

Die Wundgröße wurde durch Planimetrie bestimmt. Hierbei bedeutet:
0 = keine Beläge,
1 = 20% der Wundoberfläche waren mit Fibrinbelägen bedeckt,
2 = 40% der Wundoberfläche waren mit Fibrinbelägen bedeckt,
3 = 60% der Wundoberfläche waren mit Fibrinbelägen bedeckt,
4 = 80% der Wundoberfläche waren mit Fibrinbelägen bedeckt und
5 = 100% der Wundoberfläche waren mit Fibrinbelägen bedeckt.

Das Granulationsgewebe wurde ähnlich quantifiziert:
0 kein Granulationsgewebe erkennbar,
1 = 20% der Wundoberfläche waren durch rot imponierendes Granulationsgewebe gekennzeichnet,
2 = 40% der Wundoberfläche waren durch rot imponierendes Granulationsgewebe gekennzeichnet,
3 = 60% der Wundoberfläche waren durch rot imponierendes Granulationsgewebe gekennzeichnet,
4 = 80% der Wundoberfläche waren durch rot imponierendes Granulationsgewebe gekennzeichnet und
5 = 100% der Wundoberfläche waren durch rot imponierendes Granulationsgewebe gekennzeichnet.

Somit konnte durch die Behandlung der pathologische Zustand der chronischen Wunden in einen normalen, natürlichen Heilungsverlauf überführt werden.

## Patentansprüche

1. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom, als Mittel zur Hemmung von Proteasen, wobei das Partikelgemisch a) 20 bis 98 Gew.-% Partikel mit einer Partikelgröße x mit 45 ≤ x ≤ 300 µm und b) 2 bis 80 Gew.-% Partikel mit einer Partikelgröße x mit x > 300 µm umfasst.

2. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach Anspruch 1, wobei die Behandlung Gewebeaufbau und/oder Regulation des Gewebeaufbaus umfasst.

3. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach Anspruch 1 oder 2, wobei das Partikelgemisch in einer mehrschichtigen Wundauflage (10, 20, 40) vorhanden ist, die das Partikelgemisch und ein Trägermaterial (17, 27, 47) für das Partikelgemisch umfasst, wobei das Partikelgemisch Polyacrylatpartikel (15, 16, 25, 26, 45, 46) unterschiedlicher Größe enthält, die Wundauflage weiterhin mindestens 10 Gew.-% Polyacrylatpartikel (15, 16, 25, 26, 45, 46) bezogen auf Gesamtmenge an Trägermaterial umfasst und wobei das Partikelgemisch
a) 20 bis 98 Gew.-% Partikel mit einer Partikelgröße x mit 150 < x ≤ 300 µm als Mittel zur Hemmung von Proteasen in der Wunde und
b) 2 bis 80 Gew.-% Partikel mit einer Partikelgröße x mit 600 < x ≤ 850 µm als Mittel zur Auf- und / oder Abgabe von wässrigen Lösungen enthält.

4. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-3, wobei die Polyacrylatpartikel (15, 16, 25, 26, 45, 46) höchstens 10 Gew.-% Wasser bezogen auf das Gesamtgewicht der Polyacrylatpartikel umfassen.

5. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-4, wobei die Polyacrylatpartikel (15, 16, 25, 26, 45, 46) ein vernetztes und/ oder quervernetztes und/ oder oberflächenvernetztes Polyacrylat umfassen.

6. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-5, wobei die Wundauflage weiterhin eine das Partikelgemisch und das Trägermaterial (47) für das Partikelgemisch umschließende Umhüllung (49) oder eine das Partikelgemisch und das Trägermaterial (17, 27) von einer Wunde beabstandende Wundkontaktschicht (11, 21) umfasst.

7. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-6, wobei das Trägermaterial (17, 27, 47) für die Partikel ein Fasermaterial ist, das als erste Fasern Stapelfasern aus synthetischen und/ oder natürlichen Polymeren enthält.

8. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-18, wobei die Umhüllung (49) ein Nonwoven, ein Gestrick, ein Gewirk oder ein Gewebe ist.

9. Partikelgemisch bestehend aus Polyacrylatpartikel unterschiedlicher Größe zur Verwendung bei der Behandlung von Dekubitus, Ulcus cruris venosum, Ulcus cruris arteriosum oder diabetischem Fußsyndrom nach einem der Ansprüche 1-89, wobei die Wundauflage weiterhin eine Wundkontaktschicht (11, 21) aufweist, die keine Partikel umfasst.

## Claims

1. Particle mixture consisting of polyacrylate particle of differing size for use in the treatment of pressure ulcers, venous leg ulcers, arterial leg ulcers or diabetic foot syndrome, as means for the inhibition of proteases, the particle mixture comprising a) 20 to 98% by weight of particles having a particle size x where 45 ≤ x ≤ 300 µm and b) 2 to 80% by weight of particles having a particle size x where x 〉 300 µm.

2. Particle mixture consisting of polyacrylate particle of differing size for use in the treatment of pressure ulcers, venous leg ulcers, arterial leg ulcers or diabetic foot syndrome according to Claim 1, wherein the treatment comprises tissue formation and/or regulation of the tissue formation.

3. Particle mixture consisting of polyacrylate particle of differing size for use in the treatment of pressure ulcers, venous leg ulcers, arterial leg ulcers or diabetic foot syndrome according to Claim 1 or 2, wherein the particle mixture is present in a multilayer wound dressing (10, 20, 40) comprising the particle mixture and a support material (17, 27, 47) for the particle mixture, wherein the particle mixture contains polyacrylate particles (15, 16, 25, 26, 45, 46) of differing size, the wound dressing further comprises at least 10% by weight of polyacrylate particles (15, 16, 25, 26, 45, 46) based on the total amount of support material and wherein the particle mixture contains
a. 20 to 98% by weight of particles having a particle size x where 150 〈 x ≤ 300 µm as means for the inhibition of proteases in the wound and
b. 2 to 80% by weight of particles having a particle size x where 600 〈 x ≤ 850 µm as means for the loading and/or delivery of aqueous solutions.

4. Particle mixture consisting of polyacrylate particle of differing size for use in the treatment of pressure ulcers, venous leg ulcers, arterial leg ulcers or diabetic foot syndrome according to any of Claims 1-3, wherein the polyacrylate particles (15, 16, 25, 26, 45, 46) comprise no more than 10% by-weight of water based on the total weight of the polyacrylate particles.

5. Particle mixture consisting of polyacrylate particle of differing size for use in the treatment of pressure ulcers, venous leg ulcers, arterial leg ulcers or diabetic foot syndrome according to any of Claims 1-4, wherein the polyacrylate particles (15, 16, 25, 26, 45, 46) comprise a crosslinked and/or cross-linked and/or surface-crosslinked polyacrylate.

6. Particle mixture consisting of polyacrylate particle of differing size for use in the treatment of pressure ulcers, venous leg ulcers, arterial leg ulcers or diabetic foot syndrome according to any of Claims 1-5, wherein the wound dressing further comprises a sheath (49) enclosing the particle mixture and the support material (47) for the particle mixture, or a wound contact layer (11, 21) spacing the particle mixture and the support material (17, 27) from a wound.

7. Particle mixture consisting of polyacrylate particle of differing size for use in the treatment of pressure ulcers, venous leg ulcers, arterial leg ulcers or diabetic foot syndrome according to any of Claims 1-6, wherein the support material (17, 27, 47) for the particles is a fibrous material containing, as first fibres, staple fibres composed of synthetic and/or natural polymers.

8. Particle mixture consisting of polyacrylate particle of differing size for use in the treatment of pressure ulcers, venous leg ulcers, arterial leg ulcers or diabetic foot syndrome according to any of Claims 1-7, wherein the sheath (49) is a nonwoven, a weft-knitted fabric, a warp-knitted fabric or a woven fabric.

9. Particle mixture consisting of polyacrylate particle of differing size for use in the treatment of pressure ulcers, venous leg ulcers, arterial leg ulcers or diabetic foot syndrome according to any of Claims 1-8, wherein the wound dressing further has a wound contact layer (11, 21) comprising no particles.

## Revendications

1. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique, en tant qu'agent destiné à l'inhibition de protéases, le mélange de particules comprenant a) 20 à 98 % en poids de particules ayant une taille de particule x où 45 ≤ x ≤ 300 µm et b) 2 à 80 % en poids de particules ayant une taille de particule x où x 〉 300 µm.

2. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon la revendication 1, le traitement comprenant une reconstruction tissulaire et/ou régulation de la reconstruction tissulaire.

3. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon la revendication 1 ou 2, le mélange de particules étant présent dans un pansement multicouche (10, 20, 40) qui comprend le mélange de particules et un matériau de support (17, 27, 47) pour le mélange de particules, le mélange de particules contenant des particules de polyacrylate (15, 16, 25, 26, 45, 46) de taille variable, le pansement comprenant en outre au moins 10 % en poids de particules de polyacrylate (15, 16, 25, 26, 45, 46) par rapport à la quantité totale de matériau de support et le mélange de particules contenant
a. 20 à 98 % en poids de particules ayant une taille de particule x où 150 〈 x ≤ 300 µm en tant qu'agent destiné à l'inhibition de protéases dans la plaie et
b. 2 à 80 % en poids de particules ayant une taille de particule x où 600 〈 x ≤ 850 µm en tant qu'agent destiné à l'absorption et/ou à la libération de solutions aqueuses.

4. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 3, dans lequel les particules de polyacrylate (15, 16, 25, 26, 45, 46) comprennent au maximum 10 % en poids d'eau par rapport au poids total des particules de polyacrylate.

5. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 4, dans lequel les particules de polyacrylate (15, 16, 25, 26, 45, 46) comprennent un polyacrylate réticulé et/ou réticulé transversalement et/ou réticulé en surface.

6. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 5, le pansement comprenant en outre une enveloppe (49) entourant le mélange de particules et le matériau de support (47) pour le mélange de particules ou une couche en contact avec la plaie (11, 21), espaçant d'une plaie le mélange de particules et le matériau de support (17, 27).

7. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 6, le matériau de support (17, 27, 47) pour les particules étant un matériau fibreux qui contient en tant que premières fibres des fibres coupées à base de polymères synthétiques et/ou de polymères naturels.

8. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 7, l'enveloppe (49) étant un non-tissé, un tricot, un tissu à mailles ou un tissu.

9. Mélange de particules constitué de particule de polyacrylate de taille variable, destiné à l'utilisation dans le traitement d'escarres de décubitus, d'ulcère veineux de la jambe, d'ulcère artériel de la jambe ou de syndrome du pied diabétique selon l'une quelconque des revendications 1 à 8, le pansement comportant en outre une couche en contact avec la plaie (11, 21), qui ne comprend pas de particules.
